Europäisches Patentamt

European Patent Office

Office européen des brevets

(19)

(11) **EP 1 736 140 A1**

(12) **DEMANDE DE BREVET EUROPEEN**

(43) Date de publication:
   **27.12.2006 Bulletin 2006/52**

(51) Int Cl.:
   ***A61K 8/19*** *(2006.01)*    ***A61K 8/29*** *(2006.01)*
   ***A61K 8/92*** *(2006.01)*    ***A61Q 1/02*** *(2006.01)*
   ***A61Q 1/08*** *(2006.01)*

(21) Numéro de dépôt: **06114937.3**

(22) Date de dépôt: **02.06.2006**

(84) Etats contractants désignés:
   **AT BE BG CH CY CZ DE DK EE ES FI FR GB GR
   HU IE IS IT LI LT LU LV MC NL PL PT RO SE SI
   SK TR**
   Etats d'extension désignés:
   **AL BA HR MK YU**

(30) Priorité: **23.06.2005 FR 0551727**

(71) Demandeur: **L'ORÉAL**
   **75008 Paris (FR)**

(72) Inventeurs:
   • **Delacour, Marie-Laure**
     **75013 Paris (FR)**
   • **Berry, Florence**
     **94800 Villejuif (FR)**

(74) Mandataire: **Boulard, Denis**
   **L'OREAL - D.I.P.I.**
   **25-29 Quai Aulagnier**
   **92600 Asnières (FR)**

(54) **Composition cosmétique anhydre pigmentée**

(57)   L'invention a pour objet une composition cosmétique anhydre de maquillage comprenant une huile volatile, au moins 25% en poids de pigments, un agent épaississant des huiles, la composition ayant une dureté allant de 0,04 à 0,5 Newtons. L'invention a également pour objet un kit de maquillage comprenant deux compositions de couleurs différentes notamment anhydres.

L'invention a encore pour objet l'utilisation de la composition anhydre, ou d'un mélange de deux compositions du kit de maquillage pour estomper et/ou camoufler les inhomogénéités de couleur de la peau, en particulier les taches.

EP 1 736 140 A1

**Description**

La présente invention a pour objet une composition anhydre teintée de maquillage de la peau aussi bien que du visage que du corps humain, en particulier les fonds de teint, les blushs, les fards à paupière, les correcteurs de teint.

Les compositions de maquillage permettent de camoufler les zones de la peau présentant des irrégularités comme les tâches ou les zones dépigmentées.

Lorsque la peau présente des désordres pigmentaires tels que des tâches ou des zones hypo ou hyper pigmentées (correspondant respectivement à des zones plus claires ou plus foncées de la peau), il est nécessaire que le maquillage ait une couvrance satisfaisante pour camoufler efficacement tous ces défauts. Cette couvrance doit être d'autant plus importante que les défauts sont marqués. Dans ce cas, il est nécessaire d'introduire un taux élevé de pigments.

Toutefois, avec une teneur de plus de 20% de pigments, la texture de la composition devient pâteuse et donc difficile à étaler. Le maquillage obtenu n'est pas homogène, des zones de couleurs différentes apparaissent sur la peau, rendant le maquillage inesthétique. De plus, la texture pâteuse est inconfortable pour l'utilisatrice pendant et après l'application de la composition sur la peau.

Par ailleurs, le taux élevé de pigments accentue les problèmes de stabilité de la composition lors de son stockage au cours du temps. En effet, les pigments ont tendance à sédimenter et un surnagement important d'huiles peut apparaître. La composition présente alors un déphasage et n'est plus homogène : elle ne permet pas d'obtenir un maquillage homogène.

Il existe donc un besoin de disposer de compositions de maquillage permettant de camoufler efficacement les défauts de couleurs de la peau (en particulier les tâches et les zones hypo ou hyper pigmentaires) et présentant une texture permettant une application facile sur la peau.

Le demandeur a découvert qu'une telle composition peut être obtenue en associant une huile volatile, des pigments en une teneur élevée et un agent épaississant des huiles pour ajuster la viscosité.

De façon plus précise, la présente invention a pour objet une composition anhydre de maquillage de la peau du visage ou du corps humain, permettant de couvrir efficacement et de manière homogène les défauts très marqués de la peau tout en conservant une texture crémeuse, agréable à utiliser et conduisant à un maquillage présentant une bonne tenue (notamment tenue de la couleur et tenue du camouflage des défauts de la peau) et une bonne couvrance.

L'invention a pour objet, selon un premier aspect, une composition cosmétique anhydre de maquillage comprenant :

    i) une phase huileuse comprenant de 10 à 70% en poids, par rapport au poids total de la composition, d'au moins une huile volatile ;
    ii) une phase pulvérulente comprenant au moins 25% en poids de pigments, par rapport au poids total de la composition;
    iii) au moins un agent épaississant des huiles ;
    la composition ayant une force de pénétration allant de 0,04 à 0,5 Newtons.

L'invention a encore pour objet, selon un deuxième aspect, une composition cosmétique anhydre de maquillage comprenant :

    i) une phase huileuse comprenant de 10 à 70% en poids, par rapport au poids total de la composition, d'au moins une huile volatile ;
    ii) une phase pulvérulente comprenant des pigments en une teneur telle que la composition présente un indice de couvrance supérieur ou égal à 20;
    iii) au moins un agent épaississant des huiles ;
    la composition ayant une force de pénétration allant de 0,04 à 0,5 Newtons.

L'invention a également pour objet un kit de maquillage de la peau comprenant une première et une seconde compositions de couleur différente.

L'invention a également pour objet un procédé non thérapeutique de maquillage de la peau comprenant l'application sur la peau d'une composition telle que définie précédemment, ou d'un mélange d'au moins deux compositions de couleurs différentes du kit de maquillage telles que définies précédemment.

L'invention a aussi pour objet l'utilisation d'une composition, ou d'un mélange de deux compositions de couleur différente du kit de maquillage, telles que définies précédemment pour estomper et/ou camoufler les inhomogénéités de couleur de la peau, en particulier les taches.

**Composition anhydre :**

Les compositions selon la présente invention sont des compositions anhydres.

Par composition anhydre, on entend une composition comprenant moins de 5 % en poids d'eau, par rapport au poids total de la composition, de préférence moins de 2 % en poids d'eau. Plus préférentiellement encore, la composition est exempte d'eau, l'eau n'étant pas ajoutée lors de la préparation de la composition mais correspondant à l'eau résiduelle apportée par les ingrédients mélangés.

**Force de pénétration de la composition**

Les compositions selon l'invention présentent une force de pénétration allant de 0,04 à 0,5 N, de préférence allant de 0,05 à 0,3 N, de préférence encore allant de 0,07 à 0,25 N et plus préférentiellement de 0,1 à 0,2 N.

La force de pénétration (F) est la caractéristique de texture de la composition en relation avec la force nécessaire pour obtenir une déformation ou une pénétration donnée de la composition. La dureté d de la composition est mesurée en grammes avec le texturomètre. On calcule la force de pénétration en newtons selon la formule F= d × g (avec F en Newton, d en Kg et g en m/s²). En considérant l'accélération de la terre g = 10 m/s², et en exprimant la dureté en grammes, la force de pénétration est déterminée selon la relation : F = d / 100 (avec F en Newton et d en g).

La force de pénétration de la composition est mesurée à l'aide d'un texturomètre TAXT2i par la société RHEO.

La composition est conditionnée dans une capsule en inox de géométrie cylindrique, de diamètre de 60 mm et de hauteur 20 mm ou dans un pot en verre de géométrie cylindrique de diamètre 36mm et de hauteur 20 mm.

On remplit en excès la capsule ou le pot avec la composition et on arase la surface pour qu'elle soit bien lisse. On recouvre l'échantillon avec un verre de montre afin que les volatiles ne s'évaporent pas.

On mesure la force de pénétration après un stockage de 24h pendant 10 minutes à 20°C de la composition conditionnée.

Le mobile de mesure est un cône en plexiglas d'angle 40°.

La force de déclenchement du mobile est de 0,02 N. Le mobile descend vers la composition conditionnée avec une vitesse de 2mm/s. Le mobile pénètre dans la composition avec une vitesse de pénétration est de 1 mm/s et une profondeur de pénétration de 5 mm.

Une fois la profondeur de pénétration atteinte par le mobile, celui-ci retrouve son état initial avec une vitesse de 2 mm/s.

Trois mesures sont effectuées pour une même composition à trois endroits différents, régulièrement répartis et espacés au sein de l'échantillon. La moyenne de ces trois mesures indique la force de pénétration de la composition.

**Indice de couvrance de la composition**

Selon un aspect de l'invention, la composition présente un indice de couvrance supérieur à 20, de préférence supérieur à 30, et de préférence encore supérieur à 100.

L'indice de couvrance de la composition selon l'invention est mesuré selon la méthode suivante :

La composition est étalée avec une épaisseur de 30 $\mu$m sur une carte de contraste Erichsen, type 24/5, présentant un fond noir et un fond blanc, et l'on mesure la couleur à l'aide d'un colorimètre, par exemple de référence commerciale CR-300 de la société Minolta.

On mesure la couleur de la composition appliquée sur la carte de contraste de la façon suivante :

Les valeurs $L^*_{noir}$ $a^*_{noir}$ et $b^*_{noir}$ d'une part, et les valeurs $L^*_{blanc}$ $a^*_{blanc}$ et $b^*_{blanc}$ de la composition appliquée sur la carte de contraste sont mesurées dans l'espace colorimétrique CIE à l'aide d'un colorimètre CR-300.

La différence $\Delta E$ entre la couleur du dépôt sur la partie blanche de la carte de contraste et la couleur du dépôt sur la partie blanche de la carte de contraste est donnée par :

$$\Delta E = [(a^*_{noir} - a^*_{blanc})^2 + (b^*_{noir} - b^*_{blanc})^2 + (L^*_{noir} - L^*_{blanc})^2]^{1/2}.$$

Des dépôts similaires sont réalisés sur deux autres cartes de contraste et trois mesures sont effectuées sur chaque carte. La moyenne de ces 9 mesures est ensuite calculée.

L'indice de couvrance est inversement proportionnel à la variation de couleur $\Delta E$ entre les mesures sur fond noir et sur fond blanc.

L'indice de couvrance (1/ $\Delta E$)$\times$100 des compositions selon l'invention est supérieur à 20, de préférence supérieur à 30, et de préférence encore supérieur à 100.

L'indice de couvrance maximum correspond à une valeur de variation de couleur $\Delta E$ =0.

**Phase grasse**

La composition anhydre comprend une phase huileuse comprenant au moins une huile volatile.

Par phase huileuse, on entend l'ensemble des huiles présentes dans la composition.

Avantageusement, la composition comprend deux huiles volatiles de volatilité différente.

Par « huile volatile », on entend au sens de l'invention toute huile susceptible de s'évaporer au contact de la peau, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40.000 Pa (0,001 à 300 mm de Hg) et de préférence allant de 1,3 à 1300 Pa (0,01 à 10 mm de Hg).

L'huile volatile peut être choisie parmi les huiles volatiles hydrocarbonées, les huiles volatiles siliconées, les huiles volatiles fluorées, et leurs mélanges.

Selon un mode préféré de réalisation, l'huile volatile est choisie parmi les huiles hydrocarbonées.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isohexadécane, et par exemple les huiles vendues sous les noms commerciaux d'Isopars® ou de Permethyls®.

Comme huiles volatiles, on peut aussi utiliser les silicones volatiles, comme par exemple les huiles de silicones linéaires ou cycliques volatiles, notamment celles ayant une viscosité $\leq$ 5 centistokes (5 x $10^{-6}$ $m^2$/s), et ayant notamment de 2 à 10 atomes de silicium, de préférence de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile fluorée n'a généralement pas de point éclair.

Comme huile volatile fluorée, on peut citer le nonafluoroéthoxybutane, le nonafluorométhoxybutane, le décafluoropentane, le tétradécafluorohexane, le dodécafluoropentane, et leurs mélanges.

Avantageusement, la composition comprend au moins deux huiles volatiles hydrocarbonée et notamment un mélange d'isododécane et d'isohexadécane.

L'huile volatile peut être présente dans la composition selon l'invention en une teneur allant de 10 % à 70 % en poids, par rapport au poids total de la composition, de préférence allant de 10 % à 60 % en poids, préférentiellement allant de 10 % à 40 % en poids, et plus préférentiellement allant de 15 % à 30 % en poids.

La composition selon l'invention peut comprendre au moins une huile non volatile.

Par "huile non volatile", on entend une huile restant sur la peau à température ambiante et pression atmosphérique au moins plusieurs heures et ayant notamment une pression de vapeur inférieure à 0,13 Pa (0,01 mm de Hg) à température ambiante (25°C).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :

- les huiles hydrocarbonées d'origine animale,

- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810®, 812® et 818® par la société Dynamit Nobel,

- les éthers de synthèse ayant de 10 à 40 atomes de carbone ;

- les hydrocarbures linéaires ou ramifiés, d'origine minérale ou synthétique tels que la vaseline, les polydécènes, le polyisobutène hydrogéné tel que le Parleam ®, le squalane, les huiles de paraffine, et leurs mélanges,

- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit ≥ 10, comme par exemple l'huile de Purcellin (octanoate de cétostéaryle), le myristate d'isopropyle, le palmitate d'isopropyle, les benzoates d'alcools en $C_{12}$ à $C_{15}$, le laurate d'hexyle, l'adipate de diisopropyle, l'isononanoate d'isononyle, le néopentanoate d'isodécyl, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,

- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,

- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

Avantageusement, l'huile non volatile peut être choisie parmi les esters en $C_{12}$-$C_{36}$ tels que ceux décrits précédemment.

L'huile non volatile peut être présente dans la composition selon l'invention en une teneur allant de 1 % à 60 % en poids, par rapport au poids total de la composition, de préférence allant de 3 % à 50 % en poids, préférentiellement allant de 5 % à 35 % en poids, et plus préférentiellement allant de 10 % à 30 % en poids.

La phase huileuse de la composition selon l'invention est présente en une teneur totale allant de 10 à 70% en poids par rapport au poids total de la composition, de préférence allant de 10 % à 60 % en poids, par rapport au poids total de la composition, préférentiellement allant de 10 % à 40 % en poids, et plus préférentiellement allant de 15 % à 30 % en poids.

Les compositions selon l'invention peuvent également comprendre des corps gras autres que les huiles citées ci-dessus, comme des cires ou encore des corps gras pâteux.

Par cires on entend un corps gras solide à température ambiante.

On peut définir les corps gras pâteux à l'aide d'au moins une des propriétés physico-chimiques suivantes :

- une viscosité de 0,1 à 40 Pa.s (1 à 400 poises), mesurée à 40 °C avec un viscosimètre rotatif CONTRAVES TV équipé d'un mobile MS-r3 ou MS-r4 à la fréquence de 60 Hz,
- un point de fusion de 25-70 °C, de préférence 25-55 °C.

A titre de cires pouvant être utilisées selon l'invention, on peut citer :

- les cires d'origine animale telles que la cire d'abeilles, le spermaceti, la cire de lanoline et les dérivés de lanoline, les cires végétales telles que la cire de Carnauba, de Candellila, d'Ouricury, du Japon, le beurre de cacao ou les cires de fibres de liège ou de canne à sucre,

- les cires minérales, par exemple de paraffine, de vaseline, de lignite ou les cires microcristallines ou les ozokérites,

- les cires synthétiques parmi lesquelles les cires de polyéthylène, de polytétrafluoroéthylène et les cires obtenues par synthèse de Fisher-Tropsch,

- les cires de silicone, en particulier les polysiloxanes linéaires substitués; on peut citer, par exemple, les cires de silicone polyéther, les alkyl ou alkoxy-diméthicones ayant de 16 à 45 atomes de carbone, les alkyl méthicones comme la $C_{30}$-$C_{45}$ alkyl méthicone vendue sous la dénomination commerciale "AMS C 30" par DOW CORNING,

- les huiles hydrogénées concrètes à 25°C telles que l'huile de ricin hydrogénée, l'huile de jojoba hydrogénée, l'huile de palme hydrogénée, le suif hydrogéné, l'huile de coco hydrogénée et les esters gras concrets à 25°C comme le stéarate d'alkyle en $C_{20}$-$C_{40}$ vendu sous la dénomination commerciale "KESTER WAX K82H" par la société KOSTER KEUNEN,

- et/ou leurs mélanges.

De préférence, on utilisera les cires de polyéthylène, les cires microcristallines, les cires de carnauba, l'huile de jojoba hydrogénée, les cires de candellila, les cires d'abeilles et/ou leurs mélanges.

De préférence, les cires sont présentes en une teneur allant de 0,05 à 5% en poids, par rapport au poids total de la composition, de préférence encore de 0,1 à 4 % en poids,

Ces corps gras peuvent en particulier être choisis de manière variée par l'homme du métier afin de préparer une composition ayant les propriétés souhaitées, par exemple en consistance ou en texture.

La composition peut comprendre une phase grasse formée des huiles et des corps gras décrits précédemment, présente en une teneur allant de 10 % à 80% en poids par rapport au poids total de la composition, de préférence de 20% à 70% en poids.

**Phase pulvérulente**

La composition selon l'invention comprend une phase pulvérulente comprenant des pigments.

Par « pigments », il faut comprendre des particules, minérales ou organiques, insolubles dans la phase organique liquide, destinées à colorer et/ou opacifier la composition.

Les pigments peuvent être des pigments minéraux ou organiques. Comme pigments, on peut utiliser les oxydes métalliques comme les oxydes de fer (notamment ceux de couleur jaune, rouge, brun, noir), les dioxydes de titane, l'oxyde de cérium, l'oxyde de zirconium, l'oxyde de chrome ; le violet de manganèse, l'ultramarine bleue, le bleu de prusse, le bleu outremer, le bleu ferrique, l'oxychlorure de bismuth, la nacre, le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth, et leurs mélanges.

On utilise de préférence des pigments d'oxydes de fer ou de dioxyde de titane.

Les pigments peuvent être traités avec un agent hydrophobe pour les rendre compatible avec la phase organique de la composition. L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl si lanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium.

L'acide aminé peut être par exemple la lysine, l'acide glutamique, l'alanine

Le terme alkyl mentionné dans les composés cités précédemment désigne notamment un groupe alkyle ayant de 1 à 30 atomes de carbone, de préférence ayant de 5 à 16 atomes de carbone.

Des pigments traités hydrophobes sont notamment décrits dans la demande EP-A-1086683.

Les pigments sont présents, dans la composition selon l'invention, en une teneur supérieure ou égale à 25% en poids, par rapport au poids total de la composition, en particulier allant de 25% à 65% en poids, préférentiellement allant de 25% à 60% en poids, plus préférentiellement allant de 25 à 55% en poids, et en particulier allant de 30 % à 55% en poids.

Outre les pigments, la composition selon l'invention peut comprendre des charges, des nacres et/ou des colorants liposolubles.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique (par exemple feuillet, cubique, hexagonale, orthorombique, etc) . On peut citer le talc, le mica, la silice, le kaolin, les poudres de polyamide (Nylon®), les poudres de poly-β-alanine, les poudres de polyéthylène, les polyméthacrylates de métyle, les poudres de polyuréthane telle que la poudre de copolymère de diisocyanate d'hexaméthylène et de triméthylol hexyl lactone vendue sous les dénominations PLASTIC POWDER D-400 par la société TOSHIKI, les poudres de polymères de tétrafluoroéthylène (Téflon®), la lauroyl-lysine, l'amidon, le nitrure de bore, les copolymères de chlorure de polyvinylidène/acrylonitrile, de copolymères d'acide acrylique, les poudres de résine de silicone, en particulier les poudres de silsesquioxane (poudres de résine de silicone notamment décrites dans le brevet EP 293795 ; Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; le sulfate de baryum et leurs mélanges.

Selon un mode préféré de réalisation de l'invention, les compositions comprennent au moins une charge présentant une densité inférieure à 0,2 g/cm$^3$.

En particulier, la densité est comprise entre 0,01 et 0,2 g/cm$^3$, de préférence entre 0,05 et 0,1 g/cm3.

Pour obtenir cette faible masse volumique, on utilise avantageusement des charges constituées de polymères ou copolymères expansés de préférence à base d'acrylonitrile et d'un monomère acrylique ou styrénique et/ou de chlorure de vinylidène.

On peut par exemple utiliser un copolymère contenant : de 0 à 60 % de motifs dérivés du chlorure de vinylidène (notamment de 0,1 à 60%), de 20 % à 90 % de motifs dérivés d'acrylonitrile et de 0 à 50 % de motifs dérivés d'un monomère acrylique ou styrénique (notamment de 0,1 à 50%), la somme des pourcentages (en poids) étant égale à 100. Le monomère acrylique est par exemple un acrylate ou méthacrylate de méthyle ou d'éthyle. Le monomère styrénique est par exemple l'α-méthyl-styrène ou le styrène.

De préférence, les charges présentant une densité inférieure à 0,2 g/cm3 utilisées dans les compositions selon la présente invention sont des particules creuses d'un copolymère expansé de chlorure de vinylidène et d'acrylonitrile ou de chlorure de vinylidène, d'acrylonitrile et de méthacrylate.

Les particules de l'invention peuvent être obtenues, par exemple, selon les procédés des brevets et demandes de brevet EP-56219, EP-348372, EP-486080, EP-320473, EP-112807 et US-3615972.

La cavité interne des particules contient en principe un gaz qui peut être de l'air, de l'azote ou un hydrocarbure comme l'isobutane ou l'isopentane.

De façon avantageuse, les charges présentant une densité inférieure à 0,2 g/cm3 ont une granulométrie allant de 1 $\mu$m à 80 $\mu$m et encore mieux allant de 10 $\mu$m à 50 $\mu$m, et encore mieux de 10 $\mu$m à 30 $\mu$m.

Les charges présentant une densité inférieure à 0,2 g/cm3 dans les compositions selon l'invention sont par exemple les microsphères de terpolymère expansé de chlorure de vinylidène, d'acrylonitrile et de méthacrylate, vendues sous la marque EXPANCEL par la société Nobel Casco sous les références 551 DE 50 (granulométrie d'environ 40 $\mu$m), 551 DE 20 (granulométrie d'environ 30 $\mu$m et masse volumique d'environ 65 kg/m$^3$), 551 DE 12 (granulométrie d'environ 12 $\mu$m), 551 DE 80 (granulométrie d'environ 80 $\mu$m), 461 DE 50 (granulométrie d'environ 50 $\mu$m). On peut aussi utiliser des microsphères formées du même terpolymère expansé ayant une granulométrie d'environ 18 $\mu$m et une masse volumique d'environ 70 kg/m$^3$, appelées ci-dessous EL 23, ou ayant une granulométrie d'environ 34 $\mu$m et une masse volumique d'environ 20 kg/m$^3$, appelées ci-dessous EL 43.

Dans les compositions de l'invention, on utilise de préférence de 0,05 % à 10 % en poids, rapport au poids total de la composition, de charges présentant une densité inférieure à 0,2 g/cm$^3$, de préférence de 0,1 % à 5 % en poids, plus préférentiellement allant de 0,1 à 3 % en poids.

Les charges peuvent être présentes dans chacune des compositions selon l'invention en une teneur totale allant de 0,05 % à 30 % en poids, par rapport au poids total de la composition, de préférence allant de 0,5 % à 15 % en poids, et préférentiellement allant de 0,5 % à 7 % en poids.

Outre les pigments et les charges, la phase particulaire de l'invention peut comprendre des nacres et/ou des colorants additionnels.

Par « nacres », il faut comprendre des particules irisées, notamment produites par certains mollusques dans leur coquille ou bien synthétisées, insolubles dans le milieu de la composition.

Les nacres peuvent être choisies parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Par « colorants liposolubles », il faut comprendre des composés généralement organiques solubles dans les corps gras comme les huiles.

Les colorants liposolubles sont par exemple le rouge Soudan, le D&C Red n° 17, le D&C Green n° 6, le β-carotène, l'huile de soja, le brun Soudan, le D&C Yellow n° 11, le D&C Violet n° 2, le D&C orange n° 5, le jaune quinoléine, le rocou, les bromoacides.

Les nacres et/ou colorants additionnels peuvent être présents dans chacune des compositions selon l'invention en une teneur allant de 0,05 % à 30 % en poids, par rapport au poids total de la composition, de préférence allant de 1 % à 20 % en poids, et préférentiellement allant de 3 % à 10 % en poids.

Selon un mode préféré de réalisation, la phase pulvérulente et la phase huileuse sont présentes dans la composition selon un rapport pondéral phase pulvérulente /phase huileuse allant de 0,3 à 2, de préférence de 0,4 à 1,8, plus préférentiellement de 0,5 à 1,5, et plus préférentiellement encore de 0,7 à 1,3.

Selon un mode particulier de réalisation, la phase huileuse et la phase pulvérulente représentant au moins 85% en poids du poids total de la composition

Selon un mode particulier de réalisation, les compositions selon l'invention sont stables pendant deux mois à 45°C.

**Agent épaississant des huiles :**

Les compositions selon l'invention comprennent au moins un agent épaississant des huiles choisi parmi les agents épaississants polymériques, les agents épaississants minéraux et leurs mélanges.

L'agent épaississant polymérique présent dans la composition selon l'invention est un polymère amorphe formé par polymérisation d'une oléfine. L'oléfine peut être notamment un monomère à insaturation éthylénique élastomérique. Comme exemple d'oléfine, on peut citer les monomères de carbure éthylénique, ayant notamment une ou deux insaturations éthylénique, ayant de 2 à 5 atomes de carbone tels que l'éthylène, le propylène, le butadiène, l'isoprène.

L'agent épaississant polymérique est capable d'épaissir ou de gélifier la phase organique de la composition. Par polymère amorphe, on entend un polymère qui n'a pas de forme cristalline. L'agent épaississant polymérique peut être également filmogène, c'est-à-dire qu'il est capable de former un film lors de son application sur la peau.

L'agent épaississant polymérique peut être notamment un copolymère dibloc, tribloc, multibloc, radial, étoile, ou leurs mélanges.

De tels agents épaississants polymériques sont décrits dans la demande US-A-2002/005562 et dans le brevet US-A-5 221 534

Avantageusement, l'agent épaississant polymérique est un copolymère bloc amorphe de styrène et d'oléfine.

L'agent épaississant polymérique est de préférence hydrogéné pour réduire les insaturations éthyléniques résiduelles après la polymérisation des monomères.

En particulier, l'agent épaississant polymérique est un copolymère, éventuellement hydrogéné, à blocs styrène et à blocs éthylène/alkylène en $C_3$-$C_4$.

Comme copolymère dibloc, de préférence hydrogéné, on peut citer les copolymères de styrène-éthylène/propylène, les copolymères de styrène-éthylène/butadiène. Des polymères diblocs sont notamment vendus sous la dénomination Kraton® G1701 E par la société Kraton Polymers.

Comme copolymère tribloc, de préférence hydrogéné, on peut citer les copolymères de styrène-éthylène/propylène-styrène, les copolymères de styrène-éthylène/butadiène-styrène, les copolymères de styrène-isoprène-styrène, les copolymères de styrène-butadiène-styrène. Des polymères triblocs sont notamment vendus sous les dénominations Kraton® G1650, Kraton® G1652, Kraton® D1101, Kraton® D1102, Kraton® D1160 par la société Kraton Polymers.

On peut également utiliser un mélange de copolymère hydrogéné tribloc styrène-butylène/éthylène-styrène et de polymère étoile hydrogéné éthylène-propylène-styrène, un tel mélange étant notamment dans l'isododécane. De tels mélanges sont par exemple vendus par la société PENRECO sous les dénominations commerciales VERSAGEL® M5960 et VERSAGEL® M5670.

Avantageusement, on utilise comme agent épaississant polymérique un copolymère dibloc tel que ceux décrits précédemment, en particulier un copolymère dibloc de styrène-éthylène/propylène.

L'agent épaississant polymérique peut être présent en une teneur allant de 0,1 % à 10 % en poids, par rapport au poids total de la composition, de préférence allant de 0,5 % à 5 % en poids, et plus préférentiellement allant de 1 % à 3 % en poids.

La composition peut également comprendre au moins un agent épaississant minéral des huiles tel qu'une argile organophile, les silices pyrogénées.

Les argiles organophiles sont des argiles modifiées par des composés chimiques rendant l'argile apte à gonfler dans les milieux huileux.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence.

Les argiles sont des silicates contenant un cation pouvant être choisi parmi les cations de calcium, de magnésium, d'aluminium, de sodium, de potassium, de lithium et leurs mélanges.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.

Ces argiles peuvent être d'origine naturelle ou synthétique. De préférence, on utilise les argiles qui sont cosmétiquement compatibles et acceptables avec les matières kératiniques comme la peau.

L'argile organophile peut être choisie parmi la montmorrilonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leurs surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :

- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

L'agent épaississant minéral des huiles peut être présent dans la composition en une teneur allant de 0,5 % à 7 % en poids, par rapport au poids total de la composition, de préférence en une teneur allant de 1 % à 5 % en poids, et préférentiellement allant de 1 % à 3 % en poids.

L'agent épaississant des huiles est présent dans la composition en une teneur totale allant de 0,1 à 10% en poids par rapport au poids total de la composition, de préférence allant de 1 à 7 % en poids, et plus préférentiellement allant de 2 à 6% en poids.

La composition selon l'invention peut comprendre au moins un autre ingrédient cosmétique usuel pouvant être choisi notamment parmi les antioxydants, les parfums, les conservateurs, les neutralisants, les tensioactifs, les filtres solaires, les vitamines, les hydratants, les composés auto-bronzants, les actifs antirides, les émollients, les actifs hydrophiles ou lipophiles, les agents anti-radicaux libres, les sequestrants, les agents filmogènes, et leurs mélanges.

La composition décrite précédemment présente une texture qui permet de mélanger facilement deux compositions conformes à l'invention et de couleurs différentes. Ainsi, il est possible de réaliser un kit de maquillage comprenant au moins une composition et plus particulièrement deux compositions selon l'invention, permettant à l'utilisatrice de préparer de nouvelles teintes obtenues selon des mélanges pondéraux variés à partir de chaque composition du kit.

L'invention a également pour objet un kit de maquillage de la peau comprenant :

i) une première composition comportant des pigments et au moins une huile volatile, la première composition ayant un angle de teinte h allant de 40° à 72°;
ii) une seconde composition de couleur différente de la première, la seconde composition comportant des pigments et au moins une huile volatile, la seconde composition ayant un angle de teinte h allant de 40° à 72°;

les première et seconde compositions ayant une différence d'angle de teinte h allant de 4° à 40°.

Selon un mode préféré de réalisation, la première composition du kit selon l'invention présente une valeur d'angle de teinte h allant de 40° à 60°, préférentiellement allant de 42° à 56°, et plus préférentiellement allant de 42° à 52°; et la seconde composition du kit selon l'invention présente une valeur d'angle de teinte h allant de 54° à 72°, préférentiellement allant de 56° à 68°, et plus préférentiellement allant de 58° à 68°.

Selon un mode particulier de réalisation, les première et seconde compositions ayant une différence d'angle de teinte h allant de préférence de 10° à 30°, et de préférence encore de 15° à 25°.

L'invention a aussi pour objet un kit de maquillage de la peau comprenant :

i) une première composition comportant des pigments et au moins une huile volatile, la première composition ayant une clarté L* compris entre 30 et 80 ;
ii) une seconde composition de couleur différente de la première, la seconde composition comportant des pigments et au moins une huile volatile, la seconde composition ayant une clarté L* compris entre 30 et 80 ;
les première et seconde compositions ayant une différence de clarté ΔL* allant de 2 à 50.

Selon un mode préféré de réalisation, la première composition du kit selon l'invention présente une valeur de clarté L* allant de 30 à 60, préférentiellement allant de 35 à 60, et plus préférentiellement allant de 40 à 60 ; et la seconde composition du kit selon l'invention présente une valeur de clarté L* allant de 60 à 80, préférentiellement allant de 60 à 76, et plus préférentiellement allant de 60 à 70.

Selon un mode particulier de réalisation, les première et seconde compositions ayant une différence de clarté ΔL* allant de préférence de 2 à 40, et de préférence encore de 20 à 30.

L'invention a encore pour objet un kit de maquillage de la peau comprenant :

i) une première composition comportant des pigments et au moins une huile volatile, la première composition ayant une saturation C* comprise entre 10 et 40;
ii) une seconde composition de couleur différente de la première, la seconde composition comportant des pigments et au moins une huile volatile, la seconde composition ayant une saturation C* comprise entre 10 et 40 ;
les première et seconde compositions ayant une différence de saturation ΔC* allant de 0,1 à 30.

Selon un mode préféré de réalisation, la première composition du kit selon l'invention présente une valeur de saturation C* allant de 10 à 25, préférentiellement allant de 15 à 25, et plus préférentiellement allant de 20 à 25 ; et la seconde composition du kit selon l'invention présente une valeur de saturation C* allant de 25 à 40, préférentiellement allant de 25 à 35, et plus préférentiellement allant de 25 à 31.

Selon un mode particulier de réalisation, les première et seconde compositions ayant une différence de saturation ΔC* allant de préférence de 5 à 25, et de préférence encore de 5 à 20.

Les compositions répondant aux paramètres colorimétriques définis précédemment sont notamment obtenues par mélange des pigments, et éventuellement des charges, nacre, colorants organiques et autres ingrédients.

Les paramètres colorimétriques d'angles de teinte h, de clarté L* et de saturation C* sont mesurés selon la méthode décrite ci-après.

La différence d'angle de teint Δh correspond à la valeur absolue de la différence entre l'angle de teint h de la première composition du kit et celle de la deuxième composition.

De même, les différences de clarté ΔL* et de saturation ΔC*, correspondent respectivement aux différence (en valeur absolue) de clarté L* et de saturation C* entre la première et la seconde composition du kit.

Selon un mode préféré de réalisation, le kit de maquillage défini précédemment est tel que l'une au moins des première et seconde compositions est une composition anhydre telle que définie dans la présente demande.

Selon un mode de réalisation encore préféré, le kit de maquillage est tel que les première et seconde compositions sont conformes aux compositions anhydres décrites dans la présente demande.

Selon un mode de réalisation préféré, les compositions sont présentées sous forme d'un kit comportant deux compartiments dans lesquels les deux compositions sont conditionnées séparément.

Les deux compartiments peuvent être délimités par des récipients séparés, notamment sous forme de tubes, conditionnés notamment dans un même packaging tel qu'un étui ou un blister.

Les compartiments peuvent être couplés l'un à l'autre via leurs bouchons respectifs, qui peuvent être formés d'une seule et même pièce. Selon cette variante, les tubes sont alignés selon un même axe et disposés de manière à avoir leurs ouvertures respectives en regard l'une de l'autre. Les compositions du kit selon l'invention peuvent être également conditionnées dans 2 flacons pompe qu'on actionne ensemble ou séparément pour permettre un meilleur dosage des deux compositions, ou encore sous forme de 2 bouillottes avec un embout en mousse ou en pinceau, pour faciliter le mélange des 2 teintes.

Préférentiellement, on choisira des tubes qui réunissent la facilité de dosage et un moindre coût.

D'autres moyens peuvent être mis en oeuvre pour coupler les compartiments l'un à l'autre, notamment par moulage à l'intérieur d'une même pièce, ou par tout autre système d'accrochage, notamment par encliquetage ou collage.

## METHODE DE MESURE DES PARAMETRES COLORIMETRIQUES D'UNE COMPOSITION DANS LA MASSE

Le matériel utilisé est le suivant :

Coupelles en acier galvanisé (30 - 40 mm X 20 -30 mm X 2 mm)
Lames de verre (75 X 25 mm)
Spectrophotomètre MINOLTA CM 3610d
Logiciel de contrôle qualité

### 1/ Préparation de l'échantillon

On remplit la coupelle avec du vrac jusqu'à ce que la composition déborde un peu.
On pose une lame de verre sur la coupelle remplie En vérifiant qu'aucune bulle d'air ne s'est formée. Il est nécessaire que l'aspect du produit sous la lame soit parfaitement lisse et homogène pour la mesure.

### 2/ Mesure de l'échantillon

Pour mesurer les paramètres colorimétriques (L*, C*, h) de l'échantillon de composition préparé, on procède à une mesure de la couleur dans la masse du produit avec un spectrocolorimètre de marque MINOLTA® de type CM 3610d en moyenne ouverture 12mm, angle de 8° et en mode réflexion spéculaire inclus.

L'invention est illustrée plus en détails par I(es) exemple(s) décrit(s) ci-après.

### Exemple 1 : fond de teint anhydre

On a préparé un fond de teint anhydre ayant la composition suivante :

- Isohéxadécane 4,2g
- Cyclopentasiloxane 15,0g
- Isododécane 5,0g
- Isononanoate d'isononyle 5,4g
- Oxydes de fer (jaunes, noirs et bruns) 9,1g
- Oxydes de titane 35,9g
- Hectorite modifiée par chlorure de di-stéaryl di-méthyl ammonium
  à 10% dans du distillat de pétrole, vendue sous la dénomination
  BENTONE Gel SS 71 V par la société ELEMENTIS 15 g
- Copolymère dibloc styrène/éthylène-propylène vendu sous la dénomination
  KRATON G1701E par la société KRATON POLYMERS 2,1g
- stéarate de sorbitane 1,3g
- Kaolin 5,0g
- polydiméthylsiloxane réticulé vendu sous la dénomination
  DC 9506 par la société DOW CORNING 0,5g
- micro-sphères de chlorure de vinylidène/acrylonitrile/PMMA à
  l'isobutane expansées vendues sous la dénomination
  EXPANCEL 551 DE 20 D60 par la société EXPANCEL 1,5g

Le rapport pondéral entre la phase pulvérulente et la phase huileuse est de 1,22.

On mélange les huiles, le Kraton G1701 E, et le stéarate de sorbitane. On disperse BENTONE Gel SS 71 V dans le gel obtenu précédemment. Puis on disperse dans ce mélange les matières pulvérulentes : dans un premier temps les matières colorantes (pigments, colorants, nacres), et ensuite les charges.

Le fond de teint obtenu a une texture souple (Force de pénétration F = 0,25N +/-0,05N), douce et s'applique facilement sur la peau avec un bon glissant.

Ce fond de teint a un indice de couvrance de 200 mesuré selon le protocole précédemment décrit
Le maquillage obtenu est homogène, confortable, laisse sur la peau un dépôt uniforme conférant un très bon camouflage des imperfections de couleur. Par ailleurs, le maquillage présente une bonne tenue de la couleur et du camouflage des défauts cutanés au cours du la journée.

## Exemple 2 : fond de teint anhydre

On prépare un fond de teint ayant la composition suivante :

- Isohéxadécane 15g
- Isododécane 12g
- Isononanoate d'isononyle 16g
- Oxydes de fer (jaunes, noirs et bruns) 5g
- Oxydes de titane 20g
- Hectorite modifiée par chlorure de di-stéaryl di-méthyl ammonium à 10% dans du distillat de pétrole, vendue sous la dénomination BENTONE Gel SS 71 V par la société ELEMENTIS 20 g
- Copolymère dibloc styrène/éthylène-propylène vendu sous la dénomination KRATON G1701E par la société KRATON POLYMERS 2.5g
- stéarate de sorbitane 1,5g
- Poudre de Nylon (orgasol 2002 EXD NAT COS 204 par la société ARKEMA) 4g
- Talc 3.5g
- micro-sphères de chlorure de vinylidène/acrylonitrile/PMMA à l'isobutane expansées vendues sous la dénomination EXPANCEL 551 DE 20 D60 par la société EXPANCEL 0,5g

Le rapport pondéral entre la phase pulvérulente et la phase huileuse est de 0,55.

Le mode de préparation est le même que celui de l'exemple 1.

Le fond de teint obtenu présente une texture souple (la force de pénétration F est comprise entre 0,04 et 0,5 N) qui s'étale bien.

Le maquillage obtenu est homogène, camoufle les imperfections de couleurs de la peau, et est confortable pour l'utilisatrice.

## Exemple 3 : Fond de teint anhydre

On a préparé un fond de teint anhydre ayant la composition suivante :

- Isohéxadécane 15.2g
- Isododécane 11.5g
- Isononanoate d'isononyle 6.4g
- Oxydes de fer (jaunes, noirs et bruns) 10,71g
- Oxydes de titane 34,29g
- Hectorite modifiée par chlorure de di-stéaryl di-méthyl ammonium à 10% dans du distillat de pétrole, vendue sous la dénomination BENTONE Gel SS 71 V par la société ELEMENTIS 15 g
- Copolymère dibloc styrène/éthylène-propylène vendu sous la dénomination KRATON G1701E par la société KRATON POLYMERS 2.1 g
- stéarate de sorbitane 1,3g
- Poudre de Nylon 2g
- micro-sphères de chlorure de vinylidène/acrylonitrile/PMMA à l'isobutane expansées vendues sous la dénomination EXPANCEL 551 DE 20 D60 par la société EXPANCEL 1,5g

Le rapport pondéral entre la phase pulvérulente et la phase huileuse est de 1,05.

Le mode de préparation est le même que celui de l'exemple 1.

Le fond de teint obtenu a une texture souple (force de pénétration F = 0,15N), douce et s'applique facilement sur la peau avec un bon glissant.

Ce fond de teint a un indice de couvrance de 200 mesuré selon le protocole précédemment décrit

Le maquillage obtenu est homogène, confortable et laisse sur la peau un dépôt uniforme conférant un très bon camouflage des imperfections de couleur et de relief. Par ailleurs, le maquillage présente une bonne tenue de la couleur et du camouflage des défauts cutanés au cours du la journée.

**Exemple 4 : Fond de teint anhydre**

On prépare un fond de teint ayant la composition suivante :

- Isohéxadécane 15g
- Isododécane 12g
- Isononanoate d'isononyle 17.5g
- Oxydes de fer (jaunes, noirs et bruns) 25g
- Oxydes de titane 5g
- Hectorite modifiée par chlorure de di-stéaryl di-méthyl ammonium à 10% dans du distillat de pétrole, vendue sous la dénomination BENTONE Gel SS 71 V par la société ELEMENTIS 18 g
- Copolymère dibloc styrène/éthylène-propylène vendu sous la dénomination KRATON G1701E par la société KRATON POLYMERS 2.5g
- stéarate de sorbitane 1,5g
- Poudre de Nylon 2g
- Silice Pyrogénée (Aerosil 200 de la société DEGUSSA-HÜLS) 0.5g
- micro-sphères de chlorure de vinylidène/acrylonitrile/PMMA expansées à l'isobutane vendues sous la dénomination EXPANCEL 551 DE 20 D60 par la société EXPANCEL 1g

Le rapport pondéral entre la phase pulvérulente et la phase huileuse est de 0,56.
Le mode de préparation est le même que celui de l'exemple 1.
Le fond de teint obtenu présente une texture souple (la force de pénétration F est comprise entre 0,04 et 0,5 N) qui s'étale bien.
Le maquillage obtenu est homogène, camoufle les imperfections de couleurs de la peau, et est confortable pour l'utilisatrice.

**Exemple 5 : Fond de teint anhydre**

On prépare un fond de teint ayant la composition suivante :

- Isohéxadécane 8.2g
- Isododécane 10g
- Isononanoate d'isononyle 8.4g
- Oxydes de fer (jaunes, noirs et bruns) 15g
- Oxydes de titane 40g
- Hectorite modifiée par chlorure de di-stéaryl di-méthyl ammonium à 10% dans du distillat de pétrole, vendue sous la dénomination BENTONE Gel SS 71 V par la société ELEMENTIS 13g
- Copolymère dibloc styrène/éthylène-propylène vendu sous la dénomination KRATON G1701E par la société KRATON POLYMERS 2.1 g
- stéarate de sorbitane 1,3g
- PMMA 2g

Le rapport pondéral entre la phase pulvérulente et la phase huileuse est de 1,5.
Le mode de préparation est le même que celui de l'exemple 1.
Le fond de teint obtenu présente une texture souple (la force de pénétration F est comprise entre 0,04 et 0,5 N) qui s'étale bien.
Le maquillage obtenu est homogène, camoufle les imperfections de couleurs de la peau, et est confortable pour l'utilisatrice.

**Exemple 6 : Fond de teint anhydre**

On prépare un fond de teint ayant la composition suivante :

- Isohéxadécane 8.2g
- Isododécane 10g

- Isononanoate d'isononyle 8.4g
- Cyclopentasiloxane 8.2g
- Oxydes de fer (jaunes, noirs et bruns) 17g
- Oxydes de titane 18g
- Hectorite modifiée par chlorure de di-stéaryl di-méthyl ammonium à 10% dans du distillat de pétrole, vendue sous la dénomination BENTONE Gel SS 71 V par la société ELEMENTIS 18g
- Copolymère dibloc styrène/éthylène-propylène vendu sous la dénomination KRATON G1701E par la société KRATON POLYMERS 2.5g
- stéarate de sorbitane 1,5g
- PMMA 2g
- Talc 3g
- Nylon 3g
- micro-sphères de chlorure de vinylidène/acrylonitrile/PMMA expansées à l'isobutane vendues sous la dénomination EXPANCEL 551 DE 20 D60 par la société EXPANCEL 0.2g

Le rapport pondéral entre la phase pulvérulente et la phase huileuse est de 0,86.

Le mode de préparation est le même que celui de l'exemple 1.

Le fond de teint obtenu présente une texture souple (la force de pénétration F est comprise entre 0,04 et 0,5 N) qui s'étale bien.

Le maquillage obtenu est homogène, camoufle les imperfections de couleurs de la peau, et est confortable pour l'utilisatrice.

## Exemple 7 : Fond de teint anhydre

On prépare un fond de teint ayant la composition suivante :

- Isohéxadécane 8.5g
- Isododécane 10g
- Isononanoate d'isononyle 11 g
- Oxydes de fer (jaunes, noirs et bruns) 15g
- Oxydes de titane 25g
- Hectorite modifiée par chlorure de di-stéaryl di-méthyl ammonium à 10% dans du distillat de pétrole, vendue sous la dénomination BENTONE Gel SS 71 V par la société ELEMENTIS 18g
- Copolymère dibloc styrène/éthylène-propylène vendu sous la dénomination KRATON G1701E par la société KRATON POLYMERS 2.5g
- stéarate de sorbitane 1,5g
- PMMA 2g
- Talc 3g
- Nylon 3g
- micro-sphères de chlorure de vinylidène/acrylonitrile/PMMA expansées à l'isobutane vendues sous la dénomination EXPANCEL 551 DE 20 D60 par la société EXPANCEL 0.2g
- silice 0.3g

Le rapport pondéral entre la phase pulvérulente et la phase huileuse est de 1,07.

Le mode de préparation est le même que celui de l'exemple 1.

Le fond de teint obtenu présente une texture souple (la force de pénétration F est comprise entre 0,04 et 0,5 N) qui s'étale bien.

Le maquillage obtenu est homogène, camoufle les imperfections de couleurs de la peau, et est confortable pour l'utilisatrice.

## Exemple 8 : Fond de teint anhydre

On prépare un fond de teint ayant la composition suivante :

- Isohéxadécane 11.5g
- Isododécane 12g
- Isononanoate d'isononyle 16g
- Oxydes de fer (jaunes, noirs et bruns) 10g
- Oxydes de titane 25g
- Hectorite modifiée par chlorure de di-stéaryl di-méthyl ammonium à 10% dans du distillat de pétrole, vendue sous la dénomination BENTONE Gel SS 71 V par la société ELEMENTIS 18g
- Copolymère dibloc styrène/éthylène-propylène vendu sous la dénomination KRATON G1701E par la société KRATON POLYMERS 2.5g
- stéarate de sorbitane 1,5g
- Nylon 2g
- micro-sphères de chlorure de vinylidène/acrylonitrile/PMMA expansées à l'isobutane vendues sous la dénomination EXPANCEL 551 DE 20 D60 par la société EXPANCEL 1.5g

Le rapport pondéral entre la phase pulvérulente et la phase huileuse est de 0,72.

Le mode de préparation est le même que celui de l'exemple 1.

Le fond de teint obtenu présente une texture souple (la force de pénétration F est comprise entre 0,04 et 0,5 N) qui s'étale bien.

Le maquillage obtenu est homogène, camoufle les imperfections de couleurs de la peau, et est confortable pour l'utilisatrice.

### Exemple 9-10-11-12-13 :

A partir de la composition de l'exemple 3, en modifiant la proportion de dioxyde de titane et des oxydes de fer jaunes, noirs et bruns, on a préparé 5 compositions anhydres ayant les compositions pigmentaires suivantes et on a mesuré leurs valeur d'angles de teinte h, de clarté L* et de saturation C*:

| % massique dans la formule | Exemple 9 Jaune moyen | Exemple 10 Rose moyen | Exemple 11 Rouge foncé | Exemple 12 Jaune Clair | Exemple 13 Jaune Foncé |
|---|---|---|---|---|---|
| Di-Oxyde de Titane | 34.29 | 37.71 | 0 | 38.52 | 0 |
| Oxyde de Fer Noir | 0.36 | 0.4 | 5.18 | 0.38 | 0.96 |
| Oxyde de Fer Jaune | 9 | 3.71 | 10.85 | 5.07 | 41.28 |
| Oxyde de Fer Brun | 1.35 | 3.18 | 28.97 | 1.03 | 2.76 |
| L*, Clareté | 64.96 | 62.69 | 40.43 | 67.92 | 51.98 |
| C*, Saturation | 23.25 | 19.75 | 21.83 | 19.21 | 30.9 |
| h, Angle de teinte | 65.09° | 47° | 42.14° | 65.4° | 65.97° |

### Exemple 14 : kits de maquillage

On a réalisé un kit de maquillage contenant la composition de l'exemple 9 (première composition) et la composition de l'exemple 10 (seconde composition).

Chaque composition est conditionnée séparément dans un tube.

On a effectué un mélange des deux compositions selon un rapport pondéral 50/50, conduisant ainsi à une composition C dont les caractéristiques colorimétriques sont les suivantes : L* = 64,02, C* = 23,40 et h = 60,26°.

### Exemple 15 : Fard à paupières

On prépare un fard à paupière ayant la composition suivante :

- Isohéxadécane 15.2g
- Isododécane 11.5g
- Isononanoate d'isononyle 6.4g
- Oxydes de fer 10g
- Oxydes de titane 5g
- Laques 5g
- Nacres (Mica / titane ou Mica/Titane/Oxydes de fer) 25g
- Hectorite modifiée par chlorure de di-stéaryl di-méthyl ammonium à 10% dans du distillat de pétrole, vendue sous la dénomination BENTONE Gel SS 71 V par la société ELEMENTIS 15 g
- Copolymère dibloc styrène/éthylène-propylène vendu sous la dénomination KRATON G1701E par la société KRATON POLYMERS 2.1g
- stéarate de sorbitane 1,3g
- Poudre de Nylon 2 g
- Talc 1,5g

Le rapport pondéral entre la phase pulvérulente et la phase huileuse est de 1,05.
Le mode de préparation de ce fard à paupière est le même que celui de l'exemple 1 de fond de teint.
Le fard à paupières obtenu présente une texture souple qui s'étale bien.
Le maquillage obtenu est homogène, présente une couleur très intense qui dure au cours de la journée, et est confortable pour l'utilisatrice.

### Exemple 16 : blush

On prépare un blush ayant la composition suivante :

- Isohéxadécane 15g
- Isododécane 12g
- Isononanoate d'isononyle 16g
- Oxydes de fer 5g
- Oxydes de titane 5g
- Laques 5g
- Nacres (Mica / titane) 10g
- Hectorite modifiée par chlorure de di-stéaryl di-méthyl ammonium à 10% dans du distillat de pétrole, vendue sous la dénomination BENTONE Gel SS 71 V par la société ELEMENTIS 20 g
- Copolymère dibloc styrène/éthylène-propylène vendu sous la dénomination KRATON G1701E par la société KRATON POLYMERS 2.5g
- stéarate de sorbitane 1,5g
- Poudre de Nylon 4g
- Talc 3.5g
- micro-sphères de chlorure de vinylidène/acrylonitrile/PMMA expansées à l'isobutane vendues sous la dénomination EXPANCEL 551 DE 20 D60 par la société EXPANCEL 0,5g

Le rapport pondéral entre la phase pulvérulente et la phase huileuse est de 0,55.
Le mode de préparation de ce blush est le même que celui de l'exemple 1 de fond de teint.
Le blush obtenu présente une texture souple qui s'étale bien.
Le maquillage obtenu est homogène, présente une couleur intense qui dure au cours de la journée, et est confortable pour l'utilisatrice.

**Revendications**

1. Composition cosmétique anhydre de maquillage comprenant :

   i) une phase huileuse comprenant de 10 à 70% en poids, par rapport au poids total de la composition, d'au moins une huile volatile ;
   ii) une phase pulvérulente comprenant au moins 25% en poids de pigments, par rapport au poids total de la composition;
   iii) au moins un agent épaississant des huiles ;
   la composition ayant une dureté allant de 0,04 à 0,5 Newtons.

2. Composition cosmétique anhydre de maquillage comprenant :

   i) une phase huileuse comprenant de 10 à 70% en poids, par rapport au poids total de la composition, d'au moins une huile volatile ;
   ii) une phase pulvérulente comprenant des pigments en une teneur telle que la composition présente un indice de couvrance supérieur ou égal à 20;
   iii) au moins un agent épaississant des huiles ;
   la composition ayant une dureté allant de 0,04 à 0,5 Newtons.

3. Composition cosmétique selon l'une quelconque des revendications 1 et 2, **caractérisée en ce qu'**elle présente une dureté allant de 0,04 à 0,5 N, de préférence allant de 0,05 à 0,3 N, de préférence encore allant de 0,07 à 0,25 N et plus préférentiellement de 0,1 à 0,2 N.

4. Composition cosmétique selon l'une quelconque des revendications 2 ou 3, **caractérisée en ce** l'indice de couvrance est supérieur à 30, de préférence supérieur à 100.

5. Composition cosmétique selon l'une quelconque des revendications précédentes **caractérisée en ce qu'**elle comprend au moins deux huiles volatiles de volatilité différente.

6. Composition cosmétique selon l'une quelconques des revendications précédentes, **caractérisée en ce que** la (ou les) huile(s) volatile(s) est (sont) choisie(s) parmi les huiles volatiles siliconées et les huiles volatiles hydrocarbonées.

7. Composition selon l'une quelconque des revendications précédentes, **caractérisée en ce que** la (ou) les huiles volatiles est (sont) choisie(s) parmi les huiles volatiles hydrocarbonées ayant de 8 à 16 atomes de carbone; les huiles de silicones linéaires ou cycliques ayant de 2 à 7 atomes de silicium, ces silicones comportant éventuellement des groupes alkyle ou alkoxy ayant de 1 à 10 atomes de carbone ; les huiles volatiles fluorées.

8. Composition selon l'une quelconque des revendications précédentes, **caractérisée en ce que** la (ou les) huile(s) volatiles est (sont) choisie(s) parmi parmi l'isododécane, l'isodécane, l'isohexadécane, le cyclopentasiloxane, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane.

9. Composition selon l'une quelconque des revendications précédentes, **caractérisée en ce qu'**elle contient au moins une huile volatile siliconée et au moins une huile volatile hydrocarbonée.

10. Composition selon l'une quelconque des revendications précédentes, **caractérisée en ce qu'**elle contient de l'isohexadécane et de l'isododécane.

11. Composition selon l'une quelconque des revendications précédentes, **caractérisée en ce que** l'huile volatile est présente en une teneur allant de 10 % à 60 % en poids, par rapport au poids total de la composition, préférentiellement allant de 10 % à 40 % en poids, et plus préférentiellement allant de 15 % à 30 % en poids.

12. Composition selon l'une quelconque des revendications précédentes, **caractérisée en ce que** la phase huileuse est présente en une teneur totale allant de 10 à 70% en poids par rapport au poids total de la composition, de préférence allant de 10 % à 60 % en poids, par rapport au poids total de la composition, préférentiellement allant de 10 % à 40 % en poids, et plus préférentiellement allant de 15 % à 30 % en poids.

**13.** Composition selon l'une quelconque des revendications précédentes, **caractérisée en ce que** les pigments sont choisis parmi des pigments minéraux.

**14.** Composition selon la revendication précédente, **caractérisée en ce que** les pigments minéraux sont choisis parmi les pigments d'oxyde de fer ou de dioxyde de titane.

**15.** Composition selon l'une quelconque des revendications précédentes, **caractérisée en ce que** les pigments sont présents en une teneur allant de 25% à 65% en poids par rapport au poids total de la composition, de préférence allant de 25% à 60% en poids, de préférence encore allant de 25 à 55% en poids et plus préférentiellement allant de 30% à 55% en poids.

**16.** Composition selon l'une quelconque des revendications précédentes, **caractérisée en ce qu'**elle comprend au moins une charge.

**17.** Composition selon l'une quelconque des revendications précédentes, **caractérisée en ce qu'**elle contient au moins une charge choisie parmi le talc, le mica, la silice, le kaolin, les poudres de polyamide, les poudres de poly-β-alanine, les poudres de polyéthylène, les polyméthacrylates de métyle, les poudres de polyuréthane, les poudres de polymères de tétrafluoroéthylène, la lauroyl-lysine, l'amidon, le nitrure de bore, les poudres de copolymère de chlorure de polyvinylidène/acrylonitrile, de copolymères d'acide acrylique, les poudres de résine de silicone, les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, le sulfate de baryum ; et leurs mélanges.

**18.** Composition cosmétique selon l'une quelconque des revendications précédentes, **caractérisée en ce qu'**elle comprend au moins une charge de densité inférieure à 0,2 g/cm$^3$.

**19.** Composition cosmétique selon la revendication précédente, **caractérisée en ce que** la charge de densité inférieure à 0,2 g/cm$^3$ est choisie parmi les microsphères creuses de chlorure de polyvinylidène/acrylonitrile, les microsphères creuses de copolymères d'acide acrylique.

**20.** Composition selon l'une quelconque des revendications 16 à 19, **caractérisée en ce que** les charges, sont présentes en une teneur totale allant de 0,05 % à 30% en poids, par rapport au poids total de la composition, de préférence allant de 0,5 % à 15% en poids et plus préférentiellement allant de 0,5% à 7% en poids.

**21.** Composition cosmétique selon l'une quelconque des revendications précédentes, **caractérisée en ce que** le rapport pondéral entre la phase pulvérulente et la phase huileuse va de 0,3 à 2, la phase huileuse et la phase pulvérulente représentant au moins 85% en poids du poids total de la composition.

**22.** composition cosmétique selon la revendication précédente, **caractérisée en ce que** le rapport pondéral entre la phase pulvérulente et la phase huileuse va 0,3 à 2, de préférence de 0,4 à 1,8, de préférence encore de 0,5 à 1,5 et plus préférentiellement de 0,7 à 1,3.

**23.** Composition selon l'une quelconque des revendications qui précèdent, **caractérisée en ce que** la phase huileuse et la phase pulvérulente représentent au total au moins 90% en poids par rapport au poids total de la composition.

**24.** Composition selon l'une quelconque des revendications précédentes, **caractérisée en ce que** l'agent épaississant des huiles est choisi parmi les agents épaississants polymériques, les agents épaississants minéraux, et leurs mélanges.

**25.** Composition selon la revendication précédente, **caractérisée par le fait que** l'agent épaississant polymérique est formé par polymérisation de monomères de carbure éthylénique, ayant notamment une ou deux insaturations éthyléniques, ayant de 2 à 5 atomes de carbone.

**26.** Composition selon les revendications 24 et 25, **caractérisée par le fait que** l'agent épaississant polymérique est formé par polymérisation d'oléfine choisie parmi l'éthylène, le propylène, le butadiène, l'isoprène.

**27.** Composition selon l'une quelconque des revendications 24 à 26, **caractérisée par le fait que** l'agent épaississant polymérique est un copolymère, éventuellement hydrogéné, à blocs styrène et à blocs éthylène/alkylène en $C_3$-$C_4$.

**28.** Composition selon l'une quelconque des revendications 24 à 27, **caractérisée par le fait que** l'agent épaississant polymérique est un mélange de copolymère hydrogéné tribloc styrène-butylène/éthylène-styrène et de polymère étoile hydrogéné styrène-propylène-styrène.

**29.** Composition selon l'une quelconque des revendications 24 à 28, **caractérisée par le fait que** l'agent épaississant polymérique est choisi parmi les copolymères dibloc de styrène-éthylène/propylène, de styrène-éthylène/butadiène, et les copolymères tribloc de styrène-éthylène/butadiène-styrène, de styrène-isoprène-styrène et de styrène-butadiène-styrène.

**30.** composition selon l'une quelconque des revendications 24 à 29, **caractérisée en ce que** l'agent épaississant polymérique est présent dans la composition en une teneur allant de 0,1 à 10% en poids par rapport au poids total de la composition, de préférence allant de 0,5 à 5% en poids, et plus préférentiellement allant de 1 à 3% en poids.

**31.** Composition selon la revendication 24, **caractérisée par le fait que** l'agent épaississant minéral des huiles est choisi parmi les argiles organophiles et les silices pyrogénées.

**32.** Composition selon l'une quelconque des revendications 24 et 31, **caractérisée par le fait que** l'agent épaississant minéral des huiles est présent en une teneur allant de 0,5 % à 7 % en poids, par rapport au poids total de la composition, de préférence en une teneur allant de 1 % à 5 % en poids, et préférentiellement allant de 1 % à 3 % en poids.

**33.** Composition selon l'une quelconque des revendications 24 à 32, **caractérisée par le fait que** l'agent épaississant des huiles est présent dans la composition en une teneur totale allant de 0,1 % à 10 % en poids, par rapport au poids total de la composition, de préférence allant de 1 % à 7 % en poids, et préférentiellement allant de 2 % à 6 % en poids.

**34.** Composition selon l'une quelconque des revendications précédentes, **caractérisée en ce qu'**elle comprend un ingrédient cosmétique choisi parmi les antioxydants, les parfums, les conservateurs, les neutralisants, les tensioactifs, les filtres solaires, les vitamines, les hydratants, les composés auto-bronzants, les actifs antirides, les émollients, les actifs hydrophiles ou lipophiles, les agents anti-radicaux libres, les sequestrants, les agents filmogènes, et leurs mélanges.

**35.** kit de maquillage de la peau comprenant :

i) une première composition comportant des pigments et au moins une huile volatile, la première composition ayant un angle de teinte h allant de 40° à 72°;
ii) une seconde composition de couleur différente de la première, la seconde composition comportant des pigments et au moins une huile volatile, la seconde composition ayant un angle de teinte h allant de 40° à 72°;
les première et seconde compositions ayant une différence d'angle de teinte Δh allant de 4° à 40°.

**36.** Kit selon la revendication précédente, **caractérisé en ce que** la première composition présente une valeur d'angle de teinte h allant de 40° à 60°, préférentiellement allant de 42° à 56°, et plus préférentiellement allant de 42° à 52°.

**37.** Kit selon la revendication 35, **caractérisé en ce que** la seconde composition du kit présente une valeur d'angle de teinte h allant de 54° à 72°, préférentiellement allant de 56° à 68°, et plus préférentiellement allant de 58° à 68°.

**38.** Kit selon l'une quelconque des revendications 35 à 37, **caractérisé en ce que** les première et seconde compositions ont une différence d'angle de teinte Δh allant de préférence de 10° à 30°, et de préférence encore de 15° à 25°.

**39.** Kit de maquillage de la peau comprenant :

i) une première composition comportant des pigments et au moins une huile volatile, la première composition ayant une clarté L* compris entre 30 et 80 ;
ii) une seconde composition de couleur différente de la première, la seconde composition comportant des pigments et au moins une huile volatile, la seconde composition ayant une clarté L* compris entre 30 et 80 ;
les première et seconde compositions ayant une différence de clarté ΔL* allant de 2 à 50.

**40.** Kit selon la revendication précédente, **caractérisé en ce que** la première composition présente une valeur de clarté L* allant de 30 à 60, préférentiellement allant de 35 à 60, et plus préférentiellement allant de 40 à 60.

**41.** Kit selon la revendication 39, **caractérisé en ce que** la seconde composition du kit présente une valeur de clarté L* allant de 60 à 80, préférentiellement allant de 60 à 76, et plus préférentiellement allant de 60 à 70.

**42.** Kit selon l'une quelconque des revendications 39 à 41, **caractérisé en ce que** les première et seconde compositions ont une différence de clarté ∆L* allant de préférence de 2 à 40, et de préférence encore de 20 à 30.

**43.** Kit de maquillage de la peau comprenant :

i) une première composition comportant des pigments et au moins une huile volatile, la première composition ayant une saturation C* compris entre 10 et 40;
ii) une seconde composition de couleur différente de la première, la seconde composition comportant des pigments et au moins une huile volatile, la seconde composition ayant une saturation C* compris entre 10 et 40 ;
les première et seconde compositions ayant une différence de saturation ∆C* allant de 0,1 à 30.

**44.** Kit selon la revendication précédente, **caractérisé en ce que** la première composition présente une valeur de saturation C* allant de 10 à 25, préférentiellement allant de 15 à 25, et plus préférentiellement allant de 20 à 25.

**45.** Kit selon la revendication 43, **caractérisé en ce que** la seconde composition du kit présente une valeur de saturation C* allant de 25 à 40, préférentiellement allant de 25 à 35, et plus préférentiellement allant de 25 à 31.

**46.** Kit selon l'une quelconque des revendications 43 à 45, **caractérisé en ce que** les première et seconde compositions ont une différence de saturation ∆C* allant de préférence de 5 à 25, et de préférence encore de 5 à 20.

**47.** Kit de maquillage selon l'une quelconque des revendications 35 à 46, **caractérisée en ce que** l'une au moins des première et seconde compositions est une composition anhydre conforme à l'une quelconque des revendications 1 à 34.

**48.** Kit de maquillage selon l'une quelconque des revendications 35 à 46, **caractérisé en ce que** les première et seconde compositions sont des compositions anhydres selon à l'une quelconque des revendications 1 à 34.

**49.** Procédé non thérapeutique de maquillage de la peau comprenant l'application sur la peau d'une composition selon l'une quelconque des revendications 1 à 34, ou d'un mélange d'au moins deux compositions de couleurs différentes selon l'une quelconque des revendications du kit de maquillage selon l'une quelconque des revendications 35 à 48.

**50.** Utilisation d'une composition selon les revendications 1 à 34, ou d'un mélange d'au moins deux compositions de couleurs différentes du kit de maquillage selon l'une quelconque des revendications 35 à 48 pour estomper et/ou camoufler les inhomogénéités de couleur de la peau, en particulier les taches.

**Office européen des brevets**

**RAPPORT DE RECHERCHE EUROPEENNE**

Numéro de la demande

EP 06 11 4937

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,P | EP 1 584 321 A (L'OREAL) 12 octobre 2005 (2005-10-12)<br><br>* revendications 1-50 * | 1-20, 23-34, 49,50 | INV.<br>A61K8/19<br>A61K8/29<br>A61K8/92<br>A61Q1/02<br>A61Q1/08 |
| X | EP 1 044 673 A (L'OREAL) 18 octobre 2000 (2000-10-18)<br><br>* revendications 1-25 *<br>* page 6, alinéa 55 - alinéa 58 *<br>* page 8, ligne 86 - ligne 87 * | 1-20, 23-34, 49,50 | |
| X | FR 2 848 822 A (L'OREAL) 25 juin 2004 (2004-06-25)<br><br>* revendications 1-38 *<br>* page 12, ligne 12 - ligne 14 *<br>* page 13, ligne 23 - ligne 18 * | 1-20, 23-34, 49,50 | |
| X | FR 2 848 821 A (L'OREAL) 25 juin 2004 (2004-06-25)<br><br>* revendications 1-17 *<br>* page 13, ligne 23 - page 14, ligne 6 *<br>* page 19, ligne 27 - page 20, ligne 32 *<br>* page 21, ligne 12 - ligne 14 *<br>* page 22, ligne 14 - page 23, ligne 15 * | 1-20, 23-34, 49,50 | DOMAINES TECHNIQUES RECHERCHES (IPC)<br><br>A61K |
| X | EP 1 382 322 A2 (OREAL [FR]) 21 janvier 2004 (2004-01-21)<br>* le document en entier * | 35-48 | |
| X | EP 1 382 323 A2 (OREAL [FR]) 21 janvier 2004 (2004-01-21)<br>* le document en entier * | 35-48 | |
| X | EP 1 410 785 A (OREAL [FR]) 21 avril 2004 (2004-04-21)<br>* le document en entier * | 35-48 | |

Le présent rapport a été établi pour toutes les revendications

| Lieu de la recherche | Date d'achèvement de la recherche | Examinateur |
|---|---|---|
| Berlin | 10 octobre 2006 | Siatou, Evangelia |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**EP 1 736 140 A1**

## ANNEXE AU RAPPORT DE RECHERCHE EUROPEENNE
## RELATIF A LA DEMANDE DE BREVET EUROPEEN NO.

EP 06 11 4937

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

10-10-2006

| Document brevet cité au rapport de recherche | | Date de publication | Membre(s) de la famille de brevet(s) | | Date de publication |
|---|---|---|---|---|---|
| EP 1584321 | A | 12-10-2005 | FR | 2868296 A1 | 07-10-2005 |
| EP 1044673 | A | 18-10-2000 | AT | 202919 T | 15-07-2001 |
| | | | DE | 60000004 D1 | 16-08-2001 |
| | | | DE | 60000004 T2 | 25-04-2002 |
| | | | ES | 2161676 T3 | 16-12-2001 |
| | | | FR | 2792194 A1 | 20-10-2000 |
| | | | US | 6361782 B1 | 26-03-2002 |
| FR 2848822 | A | 25-06-2004 | BR | 0306086 A | 17-05-2005 |
| | | | EP | 1433460 A1 | 30-06-2004 |
| | | | MX | PA04000142 A | 17-06-2005 |
| | | | ZA | 200309964 A | 26-07-2004 |
| FR 2848821 | A | 25-06-2004 | BR | 0306088 A | 17-05-2005 |
| | | | EP | 1433462 A1 | 30-06-2004 |
| | | | MX | PA04000143 A | 17-06-2005 |
| | | | ZA | 200309963 A | 16-08-2004 |
| EP 1382322 | A2 | 21-01-2004 | CN | 1481778 A | 17-03-2004 |
| | | | FR | 2842416 A1 | 23-01-2004 |
| | | | JP | 2004131484 A | 30-04-2004 |
| EP 1382323 | A2 | 21-01-2004 | CN | 1478455 A | 03-03-2004 |
| | | | FR | 2842417 A1 | 23-01-2004 |
| | | | JP | 2004161744 A | 10-06-2004 |
| EP 1410785 | A | 21-04-2004 | JP | 2004231635 A | 19-08-2004 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1086683 A
- EP 293795 A
- EP 56219 A
- EP 348372 A
- EP 486080 A
- EP 320473 A
- EP 112807 A
- US 3615972 A
- US 2002005562 A
- US 5221534 A